# EUROPEAN PATENT APPLICATION

(11) **EP 4 410 304 A1**
(43) Date of publication of application: **07.08.2024**
(21) Application number: 22873232.7
(22) Date of filing: 26.09.2022
(51) Int. Cl.: A61K 39/00, A61P 35/00

(54) **ANTI-CANCER VACCINE COMPOSITION COMPRISING PEPTIDES DERIVED FROM TUMOR-ASSOCIATED ANTIGEN, AND ADJUVANT CONSISTING OF LIPOPEPTIDE AND IMMUNOACTIVE SUBSTANCE, AND USE THEREOF**

(30) Priority: 24.09.2021 KR 20210126044
(71) Applicant: Cha Vaccine Research Institute Co., Ltd, Seongnam-si, Gyeonggi-do 13230 (KR)
(72) Inventor: YUM, Jung Sun, Seongnam-si, Gyeonggi-do 13230 (KR); AHN, Byung Cheol, Seongnam-si, Gyeonggi-do 13230 (KR); CHUN, Eunyuong, Seongnam-si, Gyeonggi-do 13230 (KR); JUNG, Eunjung, Seongnam-si, Gyeonggi-do 13230 (KR); HEO, Yoonki, Seongnam-si, Gyeonggi-do 13230 (KR); KIM, Hye-jung, Seongnam-si, Gyeonggi-do 13230 (KR)
(74) Representative: EP&C
(86) International application number: PCT/KR2022/014368
(87) International publication number: WO 2023/048530

(57) **Abstract**

The present invention pertains to: an anti-cancer vaccine composition comprising [peptides derived from a tumor-associated antigen (TAA)] and [an adjuvant consisting of a lipopeptide and an immunoactive substance]; and a use thereof. Specifically, the peptides derived from a tumor-associated antigen specifically bind to a human leukocyte antigen (HLA), a combination of the peptides having the above characteristics is mixed with the adjuvant in an optimal ratio to prepare a vaccine composition, and the vaccine composition is used for preventing or treating cancer.

## Description

### BACKGROUND OF THE INVENTION

### 1. Field of the Invention

The present invention relates to an anti-cancer vaccine composition comprising peptides derived from a tumor-associated antigen (TAA) and an adjuvant consisting of a lipopeptide and an immunoactive substance, and a use thereof. Specifically, the present invention relates to a use of a peptide that specifically binds to a human leukocyte antigen (HLA), an anti-cancer vaccine consisting of the peptide, a lipopeptide and a poly(I:C) adjuvant, and a pharmaceutical composition comprising the anti-cancer vaccine as an active ingredient as a preventive or therapeutic agent for cancer.

### 2. Description of the Related Art

Cancer is characterized by the uncontrolled cell growth compared to normal cells, the ability to invade nearby tissues, and the ability to metastasize to other distant tissues. In modern times, with the development of scientific and medical technology, many diseases have become treatable, and incurable diseases have almost disappeared. However, unlike other diseases, cancer requires a very complex and demanding treatment, and even that treatment is not completely effective.

Anti-cancer treatment is performed by physically removing the cancerous tissue, suppressing the expression of genes necessary for the survival of cancer cells, such as anti-apoptotic molecules, telomerase, growth factor receptor genes, and signaling molecules, or inducing apoptosis through irradiation or the administration of anti-cancer agents (Knudson AG, Nature reviews. Cancer 1(2): 157-162, 2001).

However, the problem with this treatment is that not only cancer cells but also normal cells can be affected, resulting in side effects. Accordingly, the need for an anti-cancer vaccine as an alternative method of treating cancer is emerging.

The anti-cancer vaccine is a vaccine made to attack cancer cells by remembering antigens unique to cancer cells in the human immune system, and there are various types of antigens and methods of antigen delivery (e.g. peptides, DNA, RNA, and dendritic cells). Unlike traditional preventive vaccines that prevent external viral or bacterial infections, vaccines for cancer treatment must induce an immune response against self-antigens rather than non-self-antigens. In addition, the vaccine should work when administered after the formation of cancer and exert an immune effect that suppresses cancer, and should present as many different types of cancer antigens as possible because the expression rate of each cancer antigen is different even in patients with the same cancer type.

In fact, many types of tumor-associated antigens are self-antigens and are immune tolerogens, so it is difficult to induce a specific immune response. These limitations can be overcome by inducing an immune response to antigens commonly expressed in various cancer types, or antigens highly expressed in one cancer type. Recently, there have been reports on antigens that may be common in various cancer types, and antigens that are highly expressed in one cancer type.

Tumor-associated antigens (TAA), which are targets of anti-cancer vaccines, are classified as follows. A) Cancer-testis antigen: Antigens belonging to this group are not expressed in normal tissues except testicular spermatocytes/spermatogonia, but are highly expressed in cancerous tissues, so they were initially called cancer-testis (CT) antigens. Since testicular cells do not express class I or II HLA molecules, these antigens cannot be recognized by T cells on normal cells and can therefore be considered immunologically tumor-specific. Well-known examples of CT antigens include MAGE members and NY-ESO-1. B) Differentiation antigen: These TAAs are proteins expressed during the differentiation of cells of a specific tissue and are antigens shared between tumors and normal cells where the tumor originates. A representative example of a differentiation antigen is Melan-A/MART-1, found in melanoma and normal melanocytes. This melanocyte lineage-associated protein is involved in the biosynthesis of melanin and, although not tumor-specific, is widely used in cancer immunotherapy. Other examples include tyrosinase for melanoma or prostate-specific antigen (PSA) for prostate cancer. C) Overexpressed TAA: These are TAAs that generally show low expression levels in normal tissues and are widely expressed in different types of tumors. Many peptide epitopes processed and potentially presented by antigen presenting cells (APCs) within normal tissues may be below the threshold level for T cell recognition and therefore do not induce an immune response in normal cells, but their overexpression in tumor cells can initiate an anti-cancer response by disrupting previously established resistance. Representative examples of TAAs in this group include Her-2/neu, survivin, telomerase, or WT-1 (Wilms' tumor 1). D) Tumor-specific antigen: These unique TAAs are caused by mutations in normal genes (β-catenin, CDK4, EGFRvlll, etc.). Some of these molecular alterations are associated with tumor transformation and/or progression. Tumor-specific antigens can generally induce a strong immune response without the risk of an autoimmune response to normal tissues. On the other hand, in most cases, tumor-specific antigens are generally not shared in many individual cancers and are expressed only in a specific cancer. E) TAA caused by abnormal post-translational modification: These TAAs can occur in proteins that are neither specific nor overexpressed, but are associated with tumors primarily by active post-translational processes. Examples of this class arise from events such as protein splicing during degradation that may or may not be tumor-specific, or, in the case of MUC-1 (Mucin 1), from altered glycosylation patterns that lead to new epitopes in the tumor.

There are methods using tumor cell lysates, proteins, DNA/RNA, plasmids, and peptides, which are T cell epitopes, as antigenic forms of anti-cancer vaccines. Among them, peptide epitopes are the most effective substances for activating antigen-specific T cells to kill cancer cells or inhibit cancer cell growth. In addition, the peptide itself has the advantages of being highly safe, inexpensive, easy to produce, and capable of combining various antigens.

Anti-cancer vaccines target peptide epitopes derived from tumor-associated antigens, which are presented by molecules of the major histocompatibility complex (MHC). In humans, the MHC molecule is referred to as HLA (human leukocyte antigen). There are two classes of MHC-molecules, MHC class I and MHC class II. MHC class I molecules consist of an α chain and a β2-microglobulin chain, while MHC class II molecules consist of an α chain and a β chain. Their three-dimensional shape forms binding grooves, which are used for non-covalent binding to peptides.

MHC class I molecules are found in most nucleated cells and present endogenous proteins, defective ribosomal products (DRIPs), and peptides resulting from proteolytic cleavage of large peptides. However, peptides derived from endosomal compartments or exogenous sources are also found on MHC class I molecules. This non-classical form of class I presentation is called cross-presentation in a literature (Brossart and Bevan, Blood, 1997).

MHC class II molecules are mostly found on professional antigen-presenting cells (APCs), which primarily present peptides of foreign or transmembrane proteins taken up by antigen-presenting cells. Complexes of peptides and MHC class I molecules are recognized by CD8+ T cells with appropriate T-cell receptor (TCR), whereas complexes of peptides and MHC class II molecules are recognized by CD4+ helper T cells with appropriate TCR. CD4+ helper T cells play an important role in effectively inducing and sustaining the response of CD8+ cytotoxic T cells. Therefore, CD4+ T cell epitopes induced by tumor-associated antigens are crucial for the development of drugs that trigger anti-tumor immune responses (Gnjatic et al., PNAS, 2003).

Helper T cells support the environment at the tumor site through cytotoxic T lymphocyte (CTL) friendly cytokines and attract effector cells, such as CTLs, natural killer cells, macrophages, and granulocytes (Tay et al., Cancer Gene Therapy, 2020). Helper T cells activated by MHC class II play an important role in regulating CTL effector functions in anti-tumor immunity. Helper T cell epitopes that cause helper T cell responses of the TH1 type support effector functions of CD8+ killer T cells include cytotoxic functions against tumor cells displaying tumor associated peptide/MHC complexes on the cell surface. In this way, the tumor-associated helper T cell peptide epitopes can be used as active pharmaceutical ingredients in vaccine compositions to stimulate anti-tumor immune responses, either alone or in combination with other tumor-associated peptides. For example, in mammalian models such as mice, even in the absence of CD8+ T lymphocytes, CD4+ T cells are known to suppress tumors through inhibition of angiogenesis by secretion of interferon gamma (IFN-g) (Beatty and Paterson, Immunologic Research, 2001; Mumberg et al., PNAS, 1999).

The identification and characterization of tumor-associated antigens recognized by CD8+ T cells (ligand: MHC class I molecule + peptide epitope) or CD4+ helper T cells (ligand: MHC class II molecule + peptide epitope) and contributed synergistically to antitumor effects by both types of CD8- and CD4-dependent responses are important for the development of tumor vaccines. Therefore, in the present invention, a peptide epitope that recognizes MHC class I molecules and a peptide epitope that recognizes MHC class II molecules were used together.

Basically, any peptide that can bind to an MHC molecule can function as a T cell epitope. A prerequisite for inducing a T cell response in vitro or in vivo is the presence of T cells with the corresponding TCR and the absence of immune tolerance to this specific epitope.

The identification of genes that are overexpressed or selectively expressed in tumor tissues or human tumor cell lines does not provide precise information about the use of antigens transcribed from these genes in immunotherapy. This is because only individual subpopulations of the epitopes of these antigens are suitable for such applications, as T cells with the corresponding TCR must be present and immune tolerance to this specific epitope must be absent or minimal. Therefore, it is important to select only overexpressed or selectively expressed peptides that appear in association with MHC molecules while minimizing T cell immune tolerance and maximizing function and proliferation.

However, because the MHC has so many alleles, it is impractical to make candidate peptides for every allele and test them directly to select those that bind strongly and stably. Instead, *in silico* algorithmic programs or databases can be used to effectively predict which peptides can bind strongly to the three-dimensional binding grooves present on various MHC molecules.

Using information about the peptide motifs involved in the binding of MHC to peptides contained in these databases, and databases obtained by eluting the peptides bound to each MHC molecule with a mass spectrometer, various peptide epitopes can be predicted and found in a given protein sequence.

Representative databases include SYFPEITHI, MHCPEP, NetMHCpan-4.1EL, and NetMHCIIpan-4.0EL. However, not all peptides predicted by the binding capacity effectively induce *in vitro* or *in vivo* T cell responses. Although the binding affinity predicted in the computer program was high, it could not actually induce sufficient immunogenicity *in vitro* or *in vivo* and could even induce immune tolerance.

Therefore, in order for these predicted peptides to be used as anti-cancer vaccines, it is necessary to select the most effective and safe peptides through practical experiments to determine whether they cause effective immunogenicity *in vitro* or *in vivo* and whether they do not induce immune tolerance. However, conducting these experiments requires a lot of effort economically, materially, and time-wise. Accordingly, in the present invention, the peptides that are known to be associated with MHC class I and II molecules in various cancer antigens, are capable of maximizing the function and proliferation of T cells, and have already been shown to be safe in various previous clinical trials were preferentially selected.

Although the binding of TCR and peptide is primarily important to increase the activation and function of T cells against tumor-associated antigens, the results of previous clinical trials using actual peptides as vaccines did not show the expected effect. The main reason is that the peptide alone did not induce sufficient immunogenicity. Effective immunogenicity requires costimulatory signaling by antigen-presenting cells and the help of appropriate cytokines. Otherwise, T cells may become anergic and immune tolerance can be induced. Therefore, an adjuvant is essential to induce appropriate and sufficient T cell activation and thereby induce an anti-cancer immune response.

In the present invention, an adjuvant consisting of a lipopeptide and an immunoactive substance is used as an anti-cancer vaccine composition. This adjuvant is an excellent inducer of humoral and cellular immune responses and increases the Th1-type immune response, which is particularly important for anti-cancer responses. Therefore, the present inventors have completed the present invention by confirming that an adjuvant composed of a lipopeptide and an immunoactive substance helps cancer antigen-specific T cell activation by the peptide and induces a more effective tumor-associated antigen-specific immune response.

### SUMMARY OF THE INVENTION

It is an object of the present invention to provide an anti-cancer vaccine composition comprising [peptides derived from a tumor-associated antigen].

It is another object of the present invention to provide an anti-cancer vaccine composition comprising [peptides derived from a tumor-associated antigen] and [an adjuvant consisting of a lipopeptide and an immunoactive substance].

It is another object of the present invention to provide a pharmaceutical composition for preventing or treating cancer comprising the above anti-cancer vaccine composition as an active ingredient.

It is another object of the present invention to provide a pharmaceutical composition for preventing or treating cancer comprising the above anti-cancer vaccine composition in combination with one or more treatments selected from the group consisting of chemotherapy, targeted therapy, immune checkpoint inhibitors, and radiation therapy.

To achieve the above objects, the present invention provides an anti-cancer vaccine composition comprising [peptides derived from one or more tumor-associated antigens selected from the group consisting of hTERT, MAGE-A3, MUC-1, Survivin, and WT-1].

The present invention also provides an anti-cancer vaccine composition comprising [peptides derived from one or more tumor-associated antigens selected from the group consisting of hTERT, MAGE-A3, MUC-1, Survivin, and WT-1] and [an adjuvant consisting of a lipopeptide and an immunoactive substance].

The [peptides derived from a tumor-associated antigen] includes one or more of the following peptides.
i) Peptide of SEQ. ID. NO: 1 derived from hTERT and of type HLA-A02 (MHC class I) (SEQ. ID. NO: 1: ILAKFLHWL),
ii) Peptide of SEQ. ID. NO: 2 derived from hTERT and of type HLA-A24 (MHC class I) (SEQ. ID. NO: 2: VYAETKHFL),
iii) Peptide of SEQ. ID. NO: 3 derived from hTERT and of type HLA-DRB1 (MHC class II) (SEQ. ID. NO: 3: EARPALLTSRLRFIPK),
iv) Peptide of SEQ. ID. NO: 4 derived from hTERT and of type HLA-DRB1 (MHC class II) (SEQ. ID. NO: 4: RPGLLGASVLGLDDI),
v) Peptide of SEQ. ID. NO: 5 derived from Survivin and of type HLA-A02 (MHC class I) (SEQ. ID. NO: 5: ELTLGEFLKL),
vi) Peptide of SEQ. ID. NO: 8 derived from Mage-A3 and of type HLA-A02 (MHC class I) (SEQ. ID. NO: 8: KVAELVHFL),
vii) Peptide of SEQ. ID. NO: 9 derived from Mage-A3 and of type HLA-A24 (MHC class I) (SEQ. ID. NO: 9: IMPKAGLLI),
viii) Peptide of SEQ. ID. NO: 12 derived from MUC-1 and of type HLA-A02 (MHC class I) (SEQ. ID. NO: 12: TAPPVHNV),
ix) Peptide of SEQ. ID. NO: 15 derived from WT-1 and of type HLA-A02 (MHC class I) (SEQ. ID. NO: 15: RMFPNAPYL), and
x) Peptide of SEQ. ID. NO: 16 derived from WT-1 and of type HLA-DRB1 (MHC class II) (SEQ. ID. NO: 16: KRYFKLSHLQMHSRKH).

The adjuvant is prepared by mixing a lipopeptide and an immunoactive substance.

The adjuvant comprises a liposome having a lipopeptide inserted into its surface and an immunoactive substance.

The liposome is composed of lipids.

The immunoactive substance is one or more selected from the group consisting of poly(I:C), QS21, MPLA, CpG, and flagellin.

The present invention also provides a pharmaceutical composition for preventing or treating cancer comprising the above anti-cancer vaccine composition as an active ingredient.

In addition, the present invention provides a pharmaceutical composition for preventing or treating cancer comprising the above anti-cancer vaccine composition in combination with one or more treatments selected from the group consisting of chemotherapy, targeted therapy, immune checkpoint inhibitors, and radiation therapy.

### ADVANTAGEOUS EFFECT

The tumor-associated antigen derived peptide provided in one aspect of the present invention can effectively induce anti-cancer immunity, and the anti-cancer vaccine composition comprising the tumor-associated antigen derived peptide and an adjuvant comprising a lipopeptide and an immunoactive substance can effectively induce anti-cancer immunity against various cancers, and the anti-cancer vaccine composition can be used in combination with conventional anti-cancer treatments such as chemical anti-cancer agents with different mechanisms, immune checkpoint inhibitors, and radiation therapy to significantly enhance the anti-cancer effect, thereby being useful for preventing and treating cancer.

### BRIEF DESCRIPTION OF THE DRAWINGS

Figure 1 is a graph showing the results of confirming the immunogenicity of the tumor-associated antigen derived single peptide provided in the present invention using ELISPOT assay.
Figure 2 is a graph showing the results of confirming the immunogenicity of the tumor-associated antigen-derived peptides provided in the present invention when combined for each antigen using ELISPOT assay.
Figure 3 is a graph showing the results of confirming the immunogenicity of the tumor-associated antigen-derived peptides provided in the present invention when combined according to the number of peptides using ELISPOT assay.
Figure 4a is a graph showing the results of comparing the frequency (%) of peptide-specific tetramer + CD8 + T cells in the groups treated with CHA-2 (IL-2 20 U/ml) peptide alone (5 ug/ml or 50 ug/ml), a combination of 10 peptides (50 ug/ml), and a combination of the peptide combination (50 ug/ml), a lipopeptide and a poly(I:C) adjuvant using tetramer binding assay.
Figure 4b is a graph showing the results of comparing the number of CHA-2 peptide-specific tetramer + CD8 + T cells through the tetramer binding assay of Figure 4a.
Figure 4c is a graph showing the results of comparing the frequency (%) of peptide-specific tetramer + CD8 + T cells in the groups treated with CHA-8 (IL-2 20 U/ml) peptide alone (5 ug/ml or 50 ug/ml), a combination of 10 peptides (50 ug/ml), and a combination of the peptide combination (50 ug/ml), a lipopeptide and a poly(I:C) adjuvant using tetramer binding assay.
Figure 4d is a graph showing the results of comparing the number of CHA-8 peptide-specific tetramer + CD8 + T cells through the tetramer binding assay of Figure 4c.
Figure 4e is a graph showing the results of comparing the frequency (%) of peptide-specific tetramer + CD8 + T cells in the groups treated with CHA-9 (IL-2 20 U/ml) peptide alone (5 ug/ml or 50 ug/ml), a combination of 10 peptides (50 ug/ml), and a combination of the peptide combination (50 ug/ml), a lipopeptide and a poly(I:C) adjuvant using tetramer binding assay.
Figure 4f is a graph showing the results of comparing the number of CHA-9 peptide-specific tetramer + CD8 + T cells through the tetramer binding assay of Figure 4f.
Figure 4g is a graph showing the results of comparing the frequency (%) of peptide-specific tetramer + CD8 + T cells in the groups treated with CHA-15 (IL-2 20 U/ml) peptide alone (5 ug/ml or 50 ug/ml), a combination of 10 peptides (50 ug/ml), and a combination of the peptide combination (50 ug/ml), a lipopeptide and a poly(I:C) adjuvant using tetramer binding assay.
Figure 4h is a graph showing the results of comparing the number of CHA-15 peptide-specific tetramer + CD8 + T cells through the tetramer binding assay of Figure 4g.
Figure 5a is a graph showing the results of confirming the cytotoxic effect of a combination of the tumor-associated antigen-derived peptides provided in the present invention in the colorectal cancer cell line SW620.
Figure 5b is a graph showing the results of Figure 5a by PBMC of three healthy individuals (Donors 8, 9, and 10).
Figure 5c is a graph showing the results of confirming the cytotoxic effect of a combination of the tumor-associated antigen-derived peptides provided in the present invention in the prostate cancer cell line PC-3.
Figure 5d is a graph showing the results of Figure 5c by PBMC of three healthy individuals (Donors 8, 9, and 10).
Figure 5e is a graph showing the results of confirming the cytotoxic effect of a combination of the tumor-associated antigen-derived peptides provided in the present invention in the breast cancer cell line MCF-7.
Figure 5f is a graph showing the results of Figure 5e by PBMC of three healthy individuals (Donors 8, 9, and 10).
Figure 5g is a graph showing the results of confirming the cytotoxic effect (CTL activity) of a combination of the tumor-associated antigen-derived peptides provided in the present invention in the ovarian cancer cell line OVCAR-3.
Figure 5h is a graph showing the results of Figure 5g by PBMC of three healthy individuals (Donors 8, 9, and 10).
Figure 6 is a graph showing the results of comparing the level of granzyme B secretion by a combination of the tumor-associated antigen-derived peptides provided in the present invention in SW620, PC-3, MCF-7, and OVCAR-3 cell lines.
Figure 7a is a graph showing the results of comparing the cytotoxic effect (CTL activity) of combinations of survivin+MAGE-A3 peptide (S+M peptide), hTERT+WT-1 peptide (H+W peptide), and 10 peptides on the colorectal cell line SW620 in Donor 8 (^{#}P<0.05 10 peptide combination group vs unstimulated group, ^{$}P<0.05 10 peptide combination group vs H+W group, ^{&}P<0.05 10 peptide combination group vs S+M group).
Figure 7b is a graph showing the results of comparing the cytotoxic effect (CTL activity) of combinations of survivin+MAGE-A3 peptide (S+M peptide), hTERT+WT-1 peptide (H+W peptide), and 10 peptides on the colorectal cell line SW620 in Donor 9 (^{#}P<0.05 10 peptide combination group vs unstimulated group, ^{$}P<0.05 10 peptide combination group vs H+W group, ^{&}P<0.05 10 peptide combination group vs S+M group).
Figure 7c is a graph showing the results of comparing the cytotoxic effect (CTL activity) of combinations of survivin+MAGE-A3 peptide (S+M peptide), hTERT+WT-1 peptide (H+W peptide), and 10 peptides on the colorectal cell line SW620 in Donor 10 (^{#}P<0.05 10 peptide combination group vs unstimulated group, ^{$}P<0.05 10 peptide combination group vs H+W group, ^{&}P<0.05 10 peptide combination group vs S+M group).
Figure 7d is a graph showing the results of comparing the level of granzyme B secretion by combinations of survivin+MAGE-A3 peptide (S+M peptide), hTERT+WT-1 peptide (H+W peptide), and 10 peptides in colorectal cancer cell line SW620 (n is the number of peptides, Donors 8-10).
Figure 8a is a graph showing the results of comparing the number of IFN-γ producing cells of the test vaccine prepared with a combination of the tumor-associated antigen-derived peptides provided in the present invention, a lipopeptide and a poly(I:C) adjuvant or another TLR adjuvant in a mouse model. [From left, PBS-negative control group, peptide treatment group, peptide + TLR2 ligand treatment group, peptide + TLR3 ligand treatment group, peptide + TLR7/8 (imiquimod) ligand treatment group, peptide + TLR9 ligand treatment (CpG) group, peptide + L-pampo treatment group].
Figure 8b is a graph showing the results of comparing the peptide-specific IFN-γ secretion levels in Figure 8a.
Figure 9 is a graph showing the results of comparing the number of IFN-γ producing cells of the test vaccines prepared with combinations of 10 peptides provided in the present invention, 10 peptides and L-pampo, and 10 peptides and Lipo-pam in a mouse model.

### DESCRIPTION OF THE PREFERRED EMBODIMENTS

Hereinafter, the present invention is described in detail.

The present invention provides an anti-cancer vaccine composition comprising [peptides derived from a tumor-associated antigen].

The tumor-associated antigen (TAA) is a protein expressed in cancer or tumor cells. Examples of TAAs include novel antigens (neoantigens expressed during tumorigenesis and altered from analogous proteins of normal or healthy cells), products of oncogenes and tumor suppressor genes, overexpressed or abnormally expressed cellular proteins (e.g. HER2, MUC-1), antigens produced by oncogenic viruses (e.g., EBV, HPV, HCV, HBV, HTLV), cancer testicular antigens (CTA, e.g., MAGE family, NY-ESO), and cell type-specific differentiation antigen (e.g. MART-1). TAA sequences can be searched experimentally or through published scientific papers or publicly available databases such as the Ludwig Institute for Cancer Research database (www.cta.lncc.br/), the Cancer Immunity database (cancerimmunity.org/peptide), and the TANTIGEN Tumor T cell antigen database (cvc.dfci.harvard.edu/tadb/).

Meanwhile, the tumor specific antigen (TSA) is an antigen produced by a specific type of tumor that does not appear in normal cells of the tissue where the tumor originated. TSAs include public antigens, neoantigens, and intrinsic antigens. Tumor-specific peptides are not expressed or minimally expressed in normal healthy cells or tissues, but are expressed at a high rate (high frequency) in subjects (in cells or tissues) with a specific disease or condition, such as a specific type of cancer. Alternatively, the tumor-specific peptide may be expressed at low levels in normal healthy cells, but at high levels in cells affected by a disease (e.g., cancer) or in a subject having the disease or condition.

The [peptides derived from a tumor-associated antigen] can be at least one selected from the group consisting of a peptide of SEQ. ID. NO: 1 derived from hTERT and of type HLA-A02 (MHC class I) (SEQ. ID. NO: 1: ILAKFLHWL), a peptide of SEQ. ID. NO: 2 derived from hTERT and of type HLA-A24 (MHC class I) (SEQ. ID. NO: 2: VYAETKHFL), a peptide of SEQ. ID. NO: 3 derived from hTERT and of type HLA-DRB1 (MHC class II) (SEQ. ID. NO: 3: EARPALLTSRLRFIPK), a peptide of SEQ. ID. NO: 4 derived from hTERT and of type HLA-DRB1 (MHC class II) (SEQ. ID. NO: 4: RPGLLGASVLGLDDI), a peptide of SEQ. ID. NO: 5 derived from survivin and of type HLA-A02 (MHC class I) (SEQ. ID. NO: 5: ELTLGEFLKL), a peptide of SEQ. ID. NO: 8 derived from Mage-A3 and of type HLA-A02 (MHC class I) (SEQ. ID. NO: 8: KVAELVHFL), a peptide of SEQ. ID. NO: 9 derived from Mage-A3 and of type HLA-A24 (MHC class I) (SEQ. ID. NO: 9: IMPKAGLLI), a peptide of SEQ. ID. NO: 12 derived from MUC-1 and of type HLA-A02 (MHC class I) (SEQ. ID. NO: 12: STAPPVHNV), a peptide of SEQ. ID. NO: 15 derived from WT-1 and of type HLA-A02 (MHC class I) (SEQ. ID. NO: 15: RMFPNAPYL), and a peptide of SEQ. ID. NO: 16 derived from WT-1 and of type HLA-DRB1 (MHC class II) (SEQ. ID. NO: 16: KRYFKLSHLQMHSRKH).

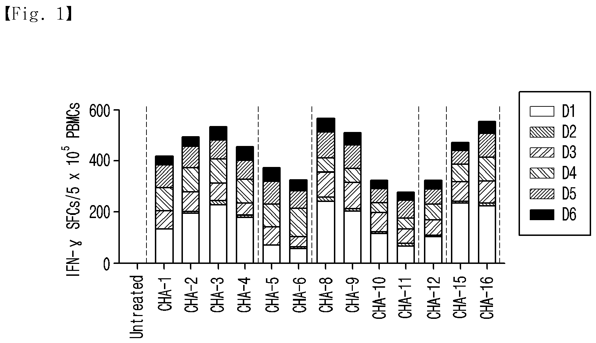

The immunogenicity of each peptide when treated to PBMCs obtained from healthy people was higher than that of the untreated control, but the degree was different (Figure 1). Based on these results, CHA-6, CHA-10, and CHA-11, which consistently showed the lowest immunogenicity, were excluded. The reason why the peptides showing low immunogenicity were removed is because immune tolerance may be induced if these peptides are added together. Therefore, among the 13 peptides, 10 peptides (SEQ. ID. NOs: 1-5, 8, 9, 12, 15 and 16) were finally confirmed based on the immunogenicity results (Table 1).

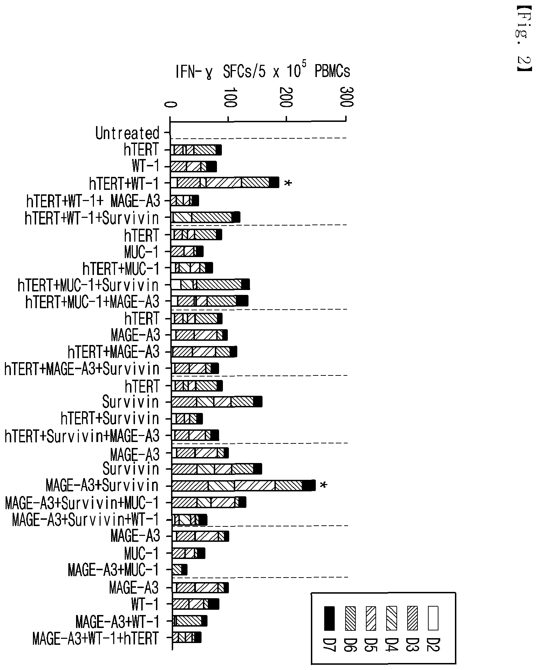

In order to select the most effective peptide combinations to induce immune responses against various tumor-associated antigens among the 10 confirmed peptides, peptides corresponding to each of the 5 antigens were first mixed and administered to PBMCs obtained from 6 healthy people, and then the increase or decrease in immunogenicity was analyzed using ELISPOT assay. As a result, it was confirmed that the immunogenicity the combination of the peptides corresponding to hTERT and WT-1 and the combination of the peptides corresponding to MAGE-A3 and survivin was effectively enhanced when each of the five antigens was mixed (Figure 2).

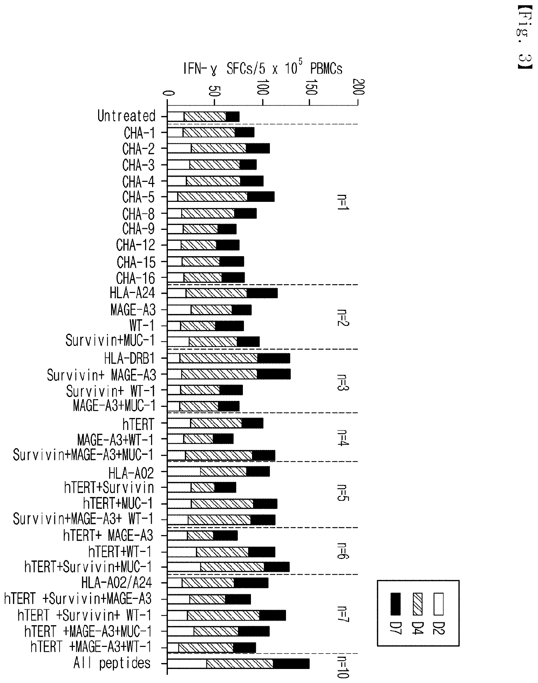

In order to examine whether the immunogenicity increases according to the combination of the number of peptides, the peptides were combined under various conditions and administered to PBMCs of three healthy people, and the ELISPOT results according to the number of peptides were displayed in a figure (Figure 3). As a result of the experiment, the group treated with the combination of 10 peptides showed higher immunogenicity than the groups treated with other combinations, and in particular, the immunogenicity was confirmed to be enhanced more than the hTERT+WT-1 combination and the survivin+MAGE-A3 combination (Figure 3).

The combination of 10 peptides (CHA-1, CHA-2, CHA-3, CHA-4, CHA-5, CHA-8, CHA-9, CHA-12, CHA-15, and CHA-16) consists of the peptides derived from various cancer antigens (hTERT, survivin, MAGE-A3, MUC1, and WT-1). The combination could induce anti-cancer immune responses against various cancer antigens, contained peptides against MHC class I and II that can activate CD8 and CD4 T cells together, which are important for anti-cancer immune responses, and provided the most enhanced immunogenicity. Therefore, the said combination was confirmed to be the most effective peptide epitope combination for a therapeutic and preventive vaccine against various cancers.

The [peptides derived from a tumor-associated antigen] and combinations thereof recognize MHC class I, MHC class II, or both.

The present invention also provides an anti-cancer vaccine composition comprising [peptides derived from a tumor-associated antigen] and [an adjuvant consisting of a lipopeptide and an immunoactive substance].

The lipopeptide may be composed of a fatty acid and several amino acids bound to a glycerol molecule. The number of amino acids constituting the fatty acid or lipopeptide in the glycerol molecule may be one or more. In this case, the fatty acid and amino acids may be chemically modified. The lipopeptide may be a lipoprotein in the form of a part of a molecule or a whole molecule derived from Gram positive or Gram negative bacteria or mycoplasma.

The lipopeptide is at least one selected from the group consisting of Pam3Cys-SKKKK, Pam3-CSKKKK, PHC-SKKKK, Ole2PamCys-Pam3Cys-SKKKK, Pam3-CSKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, Pam-CSKKKK, Dhc-SKKKK and FSL-1 (Pam2CGDPKHPKSF), but not always limited thereto.

The immunoactive substance may be any one or more selected from the group consisting of poly(I:C), QS21, monophosphoryl lipid A (MPLA), CpG, and flagellin. The poly(I:C) has been used as a strong inducer of type 1 interferon in *in vitro* and *in vivo* studies. Moreover, the poly(I:C) is known to stably and maturely form dendritic cells, the most powerful antigen-presenting cells in mammals (Rous, R. et al., International Immunol., 2004). According to these existing reports, the poly(I:C) is a strong IL-12 inducer, and IL-12 is an important immunoactive substance that induces cellular immune response and the formation of IgG2a or IgG2b by promoting the development of Th1 immune response.

The poly(I:C) may have a length of 50 to 5,000 bp. The poly(I:C) may be included in the adjuvant at a concentration of 10 to 150, 10 to 90, 10 to 50, 10 to 30, 30 to 60, 30 to 90, 30 to 150, 30 to 50, 10 to 1500, 10 to 900, 10 to 500, 10 to 300, 30 to 600, 30 to 900, 30 to 1500, or 30 to 500 µg/dose, but not always limited thereto.

The QS21 is a fraction of a saponin substance called triterpene glucoside with a molecular weight of 1990.14 Da extracted from the bark of Quillaja saponaria Molina in South America. QS21 is known to induce humoral and cellular immune responses by secreting Th1-type cytokines from antigen-presenting cells such as macrophages and dendritic cells when used with lipids such as MPLA (monophosphoryl lipid A) or cholesterol.

The QS21 may be included in the adjuvant at a concentration of 1 to 150, 1 to 90, 1 to 50, 1 to 30, 3 to 60, 3 to 90, 3 to 150, 3 to 50, 1 to 1500, 1 to 900, 1 to 500, 1 to 300, 3 to 600, 3 to 900, 3 to 1500, or 3 to 500 µg/dose, but not always limited thereto.

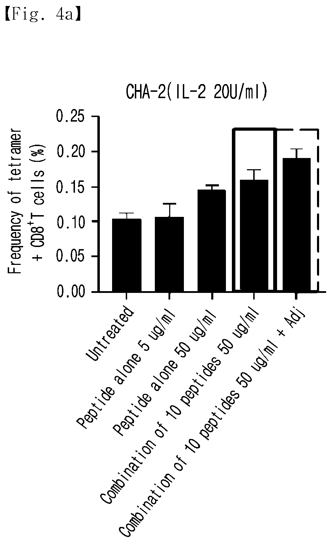

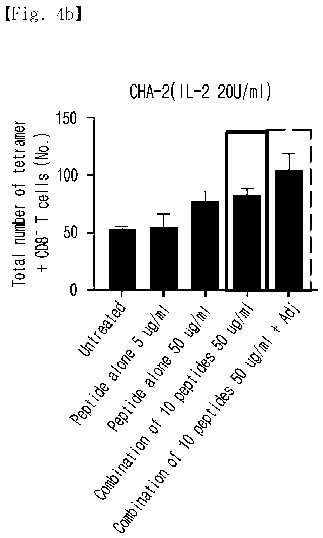

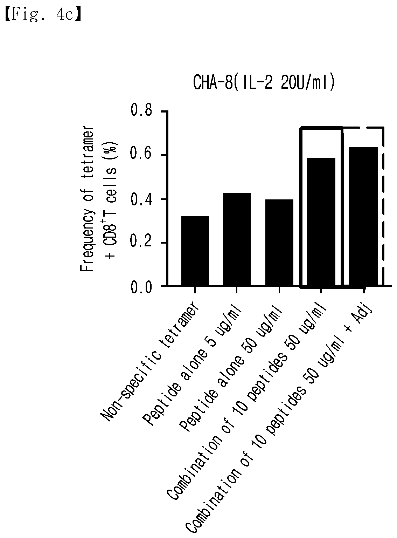

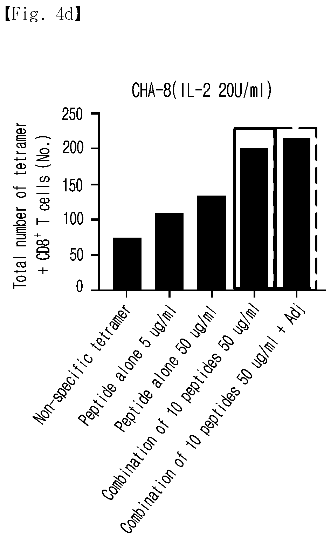

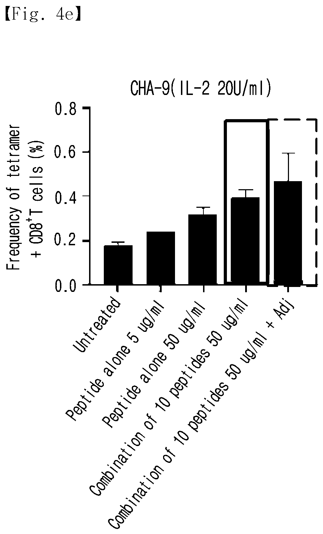

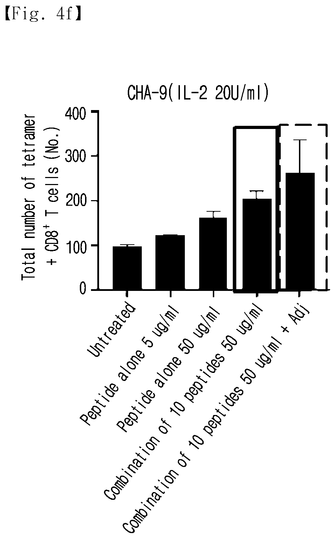

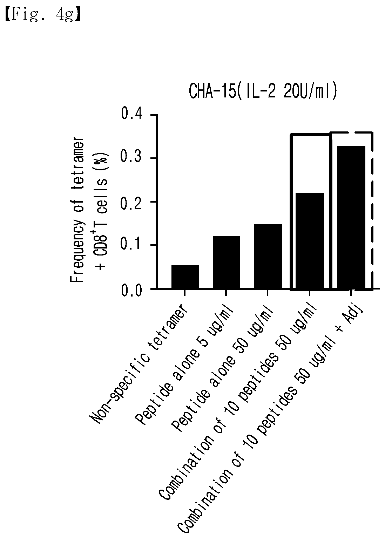

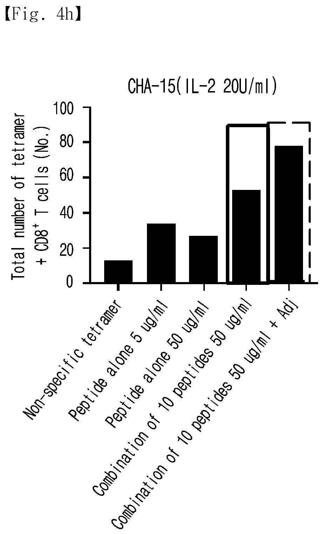

Tetramer binding assay was performed to confirm the proliferation of CD8+ T cells. When experiments were performed on the tetramerizable CHA-2, CHA-8 and CHA-9, and CHA-15 peptides, the number and frequency (%) of CD8+ T cells specific to each peptide were increased in the group treated with the combination of 10 peptides compared to each peptide alone, and the number and frequency of CD8+ T cells specific to each peptide were increased by the lipopeptide and poly(I:C) adjuvant (Figure 4).

These results show that the combination of 10 selected peptides can increase CD8+ T cells more effectively than a single peptide that can activate CD8+ T cells, and that there is a greater synergistic effect when the combination of 10 peptides was administered with an adjuvant. This is because CD4+ T cells are activated by the peptides that can bind to MHC class II (CHA-3, CHA-4, and CHA-16) in the combination of 10 peptides, and the activated CD4+ T cells in turn allow more peptide-specific CD8+ T cells to proliferate. In addition, the synergistic effect on this proliferation of CD8+ T cells can be seen to have been maximized due to the Th1 response induced by the already known lipopeptide and poly(I:C) adjuvant (Figures 4a to 4h).

To confirm whether the T cells proliferated and functionally activated by the combination of peptides derived from various tumor-associated antigens could recognize and kill cancer cells as cytotoxic T cells, CTLs, the effector cells, and cancer cells were cultured together, and then the frequency of cancer cells killed by CTLs was analyzed by flow cytometry.

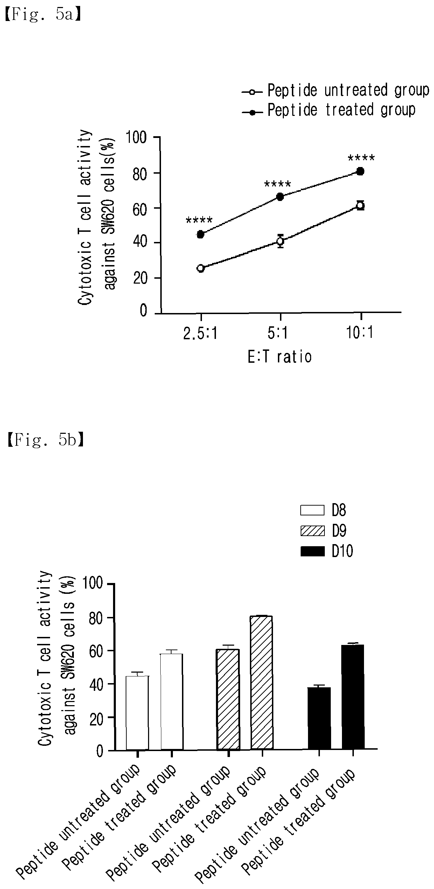

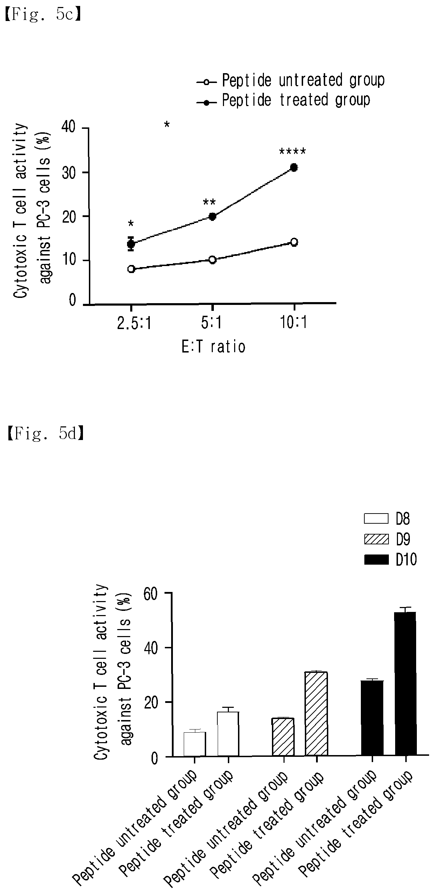

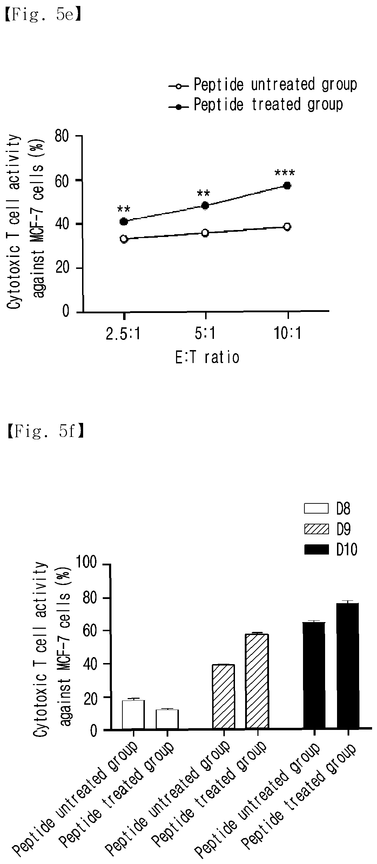

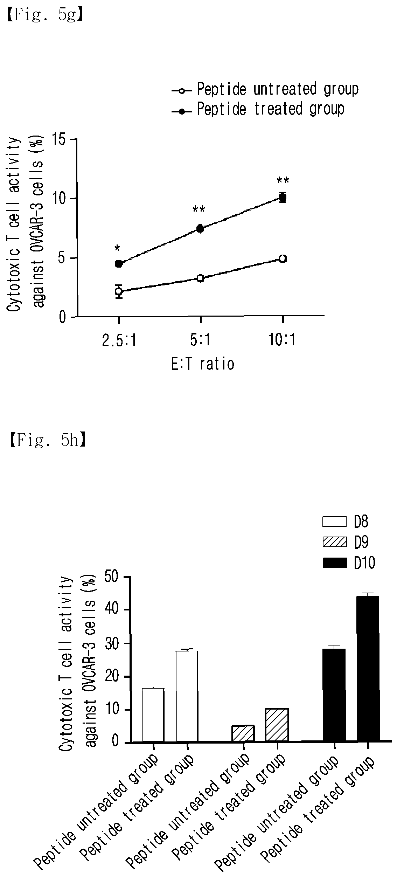

As a result of the experiment, it was confirmed that the effector cells activated by treating PBMCs of three healthy people with the peptide combination killed more cancer cells than the effector cells cultured without the peptides in the colorectal cancer cell line SW620, prostate cancer cell line PC-3, breast cancer cell line MCF-7, or ovarian cancer cell line OVCAR-3. And these results showed that it was a cell-specific cytotoxic effect, as the greater the number of effector cells, the more cancer cells were killed (Figures 5a to 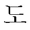 5h).

Furthermore, to confirm the activity of T cells proliferated and functionally activated by the combination of peptides derived from various tumor-associated antigens against various cancer cells, granzyme B secretion was investigated by ELISA in the culture medium in which the effector cells and cancer cells were cultured together. Granzyme B is an enzyme secreted in CD8 T cells, especially CTLs, and plays an important role in directly killing cancer cells.

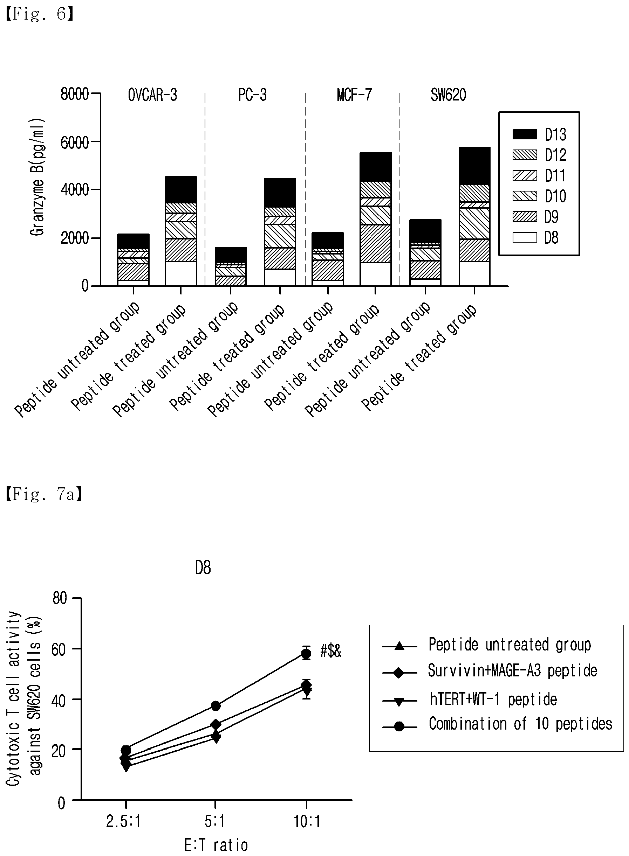

As a result of the experiment, it was confirmed that the granzyme B content was significantly higher in the culture medium in which the effector cells activated by treating PBMCs of 6 healthy people with the peptide combination and the colorectal cancer cell line SW620, prostate cancer cell line PC-3, breast cancer cell line MCF-7, or ovarian cancer cell line OVCAR-3 were cultured together than in the culture medium in which the effector cells cultured without the peptides and cancer cells were cultured together. This showed that CTLs recognized cancer cells and secreted granzyme B, which induced the death of cancer cells (Figure 6).

From the above results, it was confirmed that the tumor antigen-specific T cells proliferated and functionally activated by the peptide combination could recognize cancer cells and secrete granzyme B to directly kill cancer cells as cytotoxic T cells.

Next, the present inventors compared whether the combination of 10 peptides confirmed to exhibit excellent immunogenicity in Figures 3, 4, and 5 showed higher CTL activity efficacy than the survivin+MAGE-A3 or hTERT+WT-1 peptide combination confirmed to exhibit excellent immunogenicity in Figure 2.

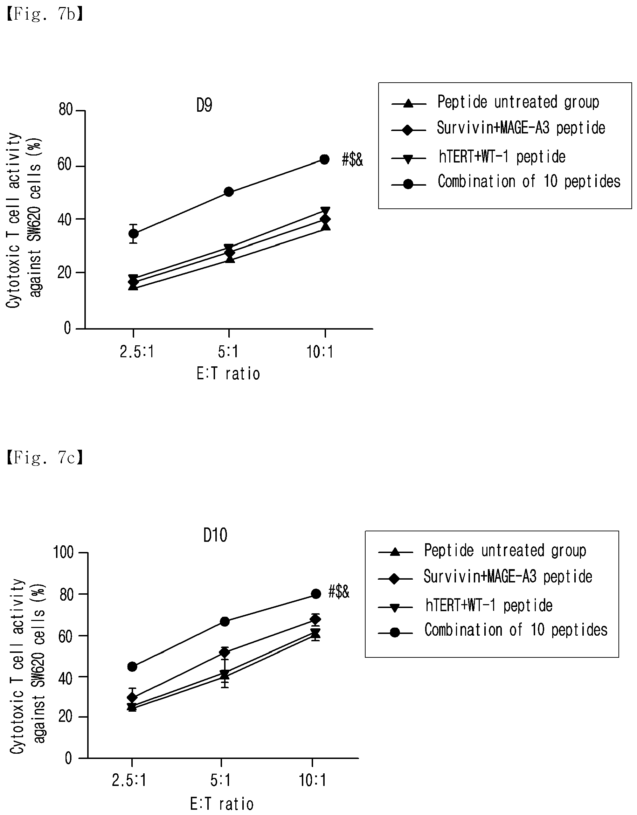

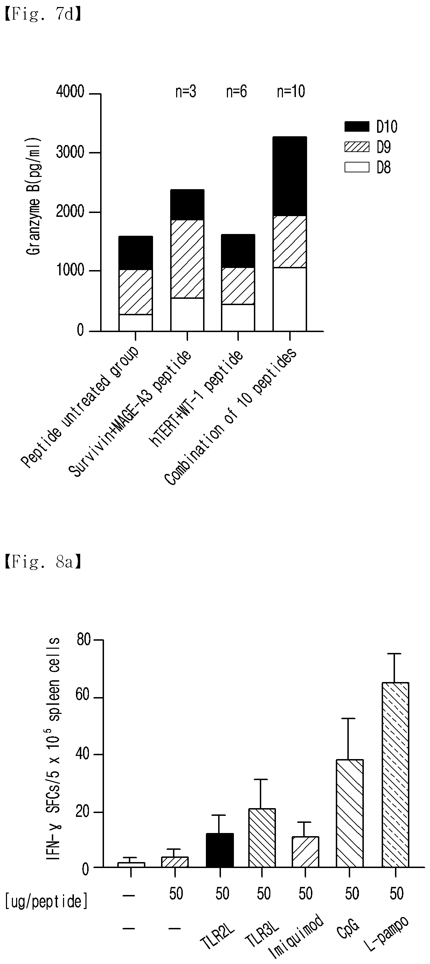

Cytotoxicity and secretion of granzyme B were analyzed in the colorectal cancer cell line SW620. 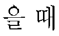 The results showed that the effector cells activated by treating PBMCs of three healthy people with the combination of 10 peptides secreted significantly higher granzyme B and were able to kill more cancer cells than the effector cells activated by treating them with the survivin+MAGE-A3 or hTERT+WT-1 peptide combination (Figures 7a to 7d).

This combination of 10 selected peptides consists of peptides derived from various tumor-associated antigens (hTERT, survivin, MAGE-A3, MUC-1, and WT-1), so it can induce anti-cancer immune responses against various cancer antigens. And the combination can activate both CD8 and CD4 T cells, which are important for anti-cancer immune responses, so that it can induce a high immune response against cancer cells and kill more cancer cells by activated CTLs.

Finally, the present inventors compared the cellular immune responses induced by the test vaccine prepared with the combination of 10 peptides and the lipopeptide and poly(I:C) adjuvant, and the test vaccines prepared with the combination of 10 peptides and the other TLR adjuvants. There are a total of 7 experimental groups as follows: PBS negative control, combination of 10 peptides, 10 peptides and TLR2 ligand, 10 peptides and TLR3 ligand, 10 peptides and TLR7/8 ligand (imiquimod), 10 peptides and TLR9 ligand (CpG), and 10 peptides and L-pampo (lipopeptide and poly (I:C) adjuvant) (Figures 8a to 8b). As a result of the experiment, it was confirmed that the test vaccine using the lipopeptide and poly(I:C) adjuvant, that is, L-pampo, significantly increased the number of cells producing IFN-γ compared to the test vaccine using the TLR2, TLR3, TLR7/8 or TLR9 adjuvant. In addition, it was confirmed that each peptide-specific IFN-γ secretion was also increased by the test vaccine using L-pampo compared to the test vaccine using the TLR2, TLR3, TLR7/8 or TLR9 adjuvant (Figures 8a to 8b).

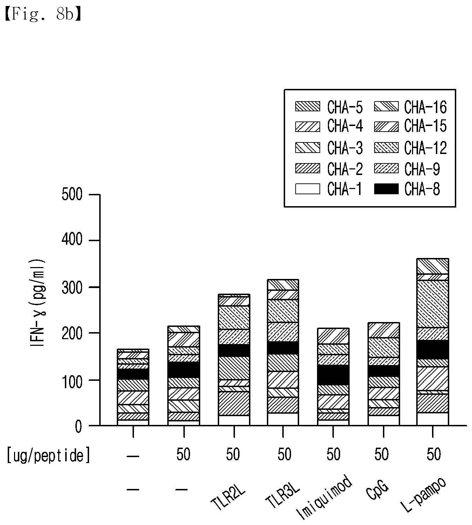

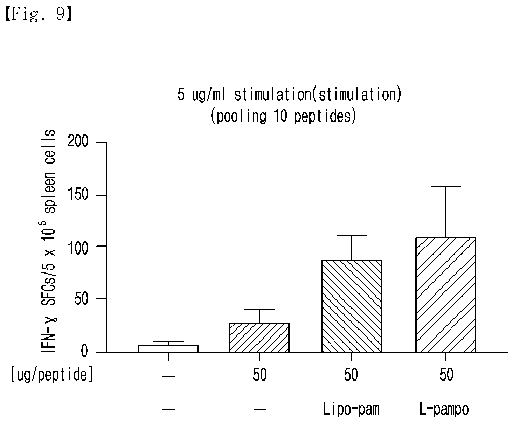

Furthermore, in the mouse model shown in Figure 8, it was determined whether the test vaccine prepared with the combination of 10 peptides and the Lipo-pam adjuvant consisting of a liposome with a lipopeptide inserted into the surface and an immunoactive substance could induce a cellular immune response similar to L-pampo. As a result, it was confirmed that the test vaccine using Lipo-pam significantly increased the number of cells producing IFN-γ compared to the control group, similar to the vaccine using L-pampo (Figure 9).

From the above results, it was confirmed that the anti-cancer vaccine composition provided in one aspect of the present invention is an anti-cancer vaccine composition that can strongly induce a cellular immune response capable of killing cancer cells compared to vaccine compositions containing other adjuvants such as TLR2, TLR3, TLR7/8, or TLR9 adjuvants.

Meanwhile, in one embodiment of the present invention, the anti-cancer vaccine composition is prepared by mixing a lipopeptide and an immunoactive substance in a certain ratio as an adjuvant.

The anti-cancer vaccine composition may be an oil emulsion or an aqueous solution formulation.

The anti-cancer vaccine composition may further comprise a buffer, an isotonic agent, a preservative, a stabilizing agent, and a solubilizing agent. The buffer may include phosphate, acetate, ammonium phosphate, ammonium carbonate, citrate, and the like.

In the composition ratio of the entire vaccine composition including the above composition, the lipopeptide is preferably included in the lipopeptide and poly(I:C) adjuvant in a weight ratio of 0.01 to 0.5 weight%, most preferably included in a weight ratio of 0.01 to 0.1 weight%, and the poly(I:C) is preferably included in a weight ratio of 0.01 to 0.4 weight, most preferably included in a weight ratio of 0.01 to 0.08 weight%, but not always limited thereto.

In addition, as an isotonic agent, sodium chloride is preferably included in a weight ratio of 0.1 to 0.8 weight%, and more preferably in a weight ratio of 0.2 to 0.6 weight%. According to a preferred embodiment of the present invention, it is most preferably included in a weight ratio of 0.44 weight%.

Furthermore, it is preferable to use phosphate, acetate, ammonium phosphate, ammonium carbonate, citrate, and the like as a buffering agent, and according to a preferred embodiment of the present invention, it is more preferable to use phosphate and acetate, but not always limited thereto. The phosphate is preferably included in a weight ratio of 0.01 to 0.4 weight%, and more preferably in a weight ratio of 0.01 to 0.2 weight%. According to a preferred embodiment of the present invention, it is most preferably included in a weight ratio of 0.13 weight%. In addition, the acetate is preferably included in a weight ratio of 0.01 to 0.3 weight%, and more preferably in a weight ratio of 0.01 to 0.2 weight%. According to a preferred embodiment of the present invention, it is most preferably included in a weight ratio of 0.08 weight%. Thus, the vaccine composition of the present invention preferably contain the buffering agent in a weight ratio of 0.02 to 0.6 weight%, and according to a preferred embodiment of the present invention, most preferably in a weight ratio of 0.15 to 0.25 weight%.

Meanwhile, in another embodiment of the present invention, the adjuvant is prepared by mixing a liposome with a lipopeptide inserted into the surface and an immunoactive substance.

The lipopeptide may be inserted into the liposome at a concentration of 20 to 250, 20 to 50, 50 to 250, 150 to 250, 50 to 150, 20 to 2500, 20 to 500, 50 to 2500, 150 to 2500, or 50 to 1500 µg/dose.

The liposome is composed of a lipid.

The lipid may be a cationic, anionic, or neutral lipid. For example, the lipid is at least one selected from the group consisting of DOTAP (1,2-dioleoyl-3-trimethylammonium-Propane), DOPE (1,2-dioleoyl-sn-glycero-3-phosphoethanolamine), DDA (dimethyldioctadecylammonium), DC-chol (3β-[N-(N',N'-dimethylaminoethane)-carbamoyl]cholesterol), DOPG (1,2-dioleoyl-sn-glycero-3-[phospho-rac-(1-glycerol)]), DPPC (1,2-dipalmitoyl-sn-glycero-3-phosphocholine), DOPC (1,2-dioleoyl-sn-glycero-3-phosphocholine) and cholesterol, but not always limited thereto.

The lipid may be inserted into the liposome at a concentration of 15 to 300, 15 to 150, 15 to 90, 15 to 50, 15 to 40, 20 to 30, 15 to 3000, 15 to 1500, 15 to 900, 15 to 500, 15 to 400, or 20 to 300 µg/dose.

The adjuvant consists of a liposome with a positive charge in which a lipopeptide with a positive charge is inserted into a lipid bilayer and an immunoactive substance with a negative charge.

The anti-cancer vaccine composition can induce both humoral and cellular immune responses.

The anti-cancer vaccine composition can enhance Th1 immune response. IgG2a or IgG2b, which promotes Th1 immune response that is effective in antiviral and anti-cancer immune responses, is produced by cytokines secreted by helper T cell 1 (Th1). Therefore, the anti-cancer vaccine composition of the present invention can also be used as a preventive or therapeutic agent for cancer that progresses from viral infection.

The humoral immune response can be induced by increasing T follicular helper (Tfh) cells and secreting cytokines by the adjuvant of the present invention.

The cellular immune response can be induced by increasing the secretions of IFN-y, TNF-α, and granzyme B.

The present invention also provides a pharmaceutical composition for preventing or treating cancer comprising the anti-cancer vaccine composition as an active ingredient.

The anti-cancer vaccine composition may have the characteristics described above.

In one embodiment of the present invention, the anti-cancer vaccine composition may include an adjuvant and an antigen. The adjuvant may include a liposome into which a lipopeptide is inserted, and may further include an immunoactive substance.

The preventive or therapeutic agent of the present invention may contain a pharmaceutically acceptable carrier, may be formulated for human or veterinary use, and can be administered through various routes. The route of administration may be oral, intraperitoneal, intravenous, intramuscular, subcutaneous, intradermal, transdermal, and intranasal, etc. Preferably, it is formulated and administered as an injection. Injections can be prepared using aqueous solvents such as physiological saline solution and Ringer's solution and non-aqueous solvents such as vegetable oil, higher fatty acid esters (e.g., ethyl oleate, etc.) and alcohols (e.g., ethanol, benzyl alcohol, propylene glycol, glycerin, etc.), and can contain pharmaceutical carriers such as stabilizers to prevent deterioration (e.g., ascorbic acid, sodium hydrogen sulfite, sodium pyrosulfite, BHA, tocopherol, EDTA, etc.), emulsifiers, buffers to adjust pH, and preservatives to inhibit microbial growth (e.g., phenyl mercury nitrate, thimerosal, benzalkonium chloride, phenol, cresol, benzyl alcohol, etc.).

The pharmaceutical composition for preventing or treating cancer of the present invention can be administered in a pharmaceutically effective amount. The term "pharmaceutically effective amount" refers to an amount that is sufficient to exhibit a vaccine effect while not causing side effects or a severe or excessive immune response, and the exact administration concentration may vary depending on the antigen to be included in the vaccine. In addition, the preventive or therapeutic agent can be easily determined by those skilled in the art according to the factors well known in the medical field, such as the patient's age, weight, health, gender, and sensitivity to the drug, administration route, and administration method. The pharmaceutical composition of the present invention can be administered once to several times.

The cancer is at least one selected from the group consisting of ovarian cancer, benign astrocytoma, malignant astrocytoma, pituitary adenoma, meningioma, cerebral lymphoma, oligodendroglioma, intracranial tumor, ependymoma, brain stem tumor, laryngeal cancer, oropharyngeal cancer, nasal cavity/paranasal sinus cancer, nasopharyngeal cancer, salivary gland cancer, hypopharyngeal cancer, thyroid cancer, oral cancer, thoracic tumor, small cell lung cancer, non-small cell lung cancer, thymic cancer, mediastinal tumor, esophageal cancer, breast cancer, abdominal tumor, stomach cancer, liver cancer, gallbladder cancer, biliary tract cancer, pancreatic cancer, small intestine cancer, colon cancer, anal cancer, bladder cancer, kidney cancer, penile cancer, prostate cancer, cervical cancer, endometrial cancer, uterine sarcoma, vaginal cancer, female external genitalia cancer, and skin cancer, but not always limited thereto.

The anti-cancer vaccine composition can be used in combination with one or more treatments selected from the group consisting of chemotherapy, targeted therapy, immune checkpoint inhibitors, and radiation therapy, and shows a synergistic anti-cancer effect compared to existing treatments.

Hereinafter, the present invention will be described in detail by the following examples and experimental examples.

However, the following examples and experimental examples are only for illustrating the present invention, and the contents of the present invention are not limited thereto.

### Example 1: Discovery of MHC class I and MHC class II antigenic peptide epitopes derived from tumor-associated antigens

### <1-1> Selection of peptide candidates for inducing immunogenicity

In order to select peptide candidates for inducing immunogenicity, the peptides derived from known tumor-associated antigens that are in clinical trials for various cancers were selected as candidates. The selected tumor-associated antigens were hTERT, survivin, MAGE-A3, MUC-1, EGFRvlll, NY-ESO-1, and WT-1 antigens, and 16 peptides derived from them were selected (Table 1).

**[Table 1]**

| | **tumor-associated antigen** | **Amino acid sequence** | **HLA class** | **HLA type** | **SEQ. ID. NO:** |
|---|---|---|---|---|---|
| CHA-1 | hTERT | ILAKFLHWL (540-548) | I | A02 | 1 |
| CHA-2 | hTERT | VYAETKHFL (324-332) | I | A24 | 2 |
| CHA-3 | hTERT | EARPALLTSRLRFIPK (611-626) | II | DRB1 | 3 |
| CHA-4 | hTERT | RPGLLGASVLGLDDI (672-686) | II | DRB1 | 4 |
| CHA-5 | Survivin | ELTLGEFLKL (96-104) | I | A02 | 5 |
| CHA-6 | Survivin | LMLGEFLKL (97-104) | I | A02 | 6 |
| CHA-7 | Survivin | AYACNTSTL (80-88) | I | A24 | 7 |
| CHA-8 | Mage-A3 | KVAELVHFL (112-120) | I | A02 | 8 |
| CHA-9 | Mage-A3 | IMPKAGLLI (195-203) | I | A24 | 9 |
| CHA-10 | Mage-A3 | TQHFVQENYLEY (246-260) | II | DRB1 | 10 |
| CHA-11 | Mage-A3 | FLWGPRALV (271-279) | I | A02 | 11 |
| CHA-12 | MUC-1 | STAPPVHNV (179-187) | I | A02 | 12 |
| CHA-13 | EGFRvIII | LEEKKGNYVVTDHC (1-13) | II | DRB1 | 13 |
| CHA-14 | NY-EOS-1 | SLLMWITQCFLPVF (157-170) | II | DRB1 | 14 |
| CHA-15 | WT-1 | RMFPNAPYL (126-134) | I | A02 | 15 |
| CHA-16 | WT-1 | KRYFKLSHLQMHSRKH (332-346) | II | DRB1 | 16 |

Among these 16 peptides, three peptides (CHA-7, CHA-13 and CHA-14) were excluded because they all had cysteine in their amino acid residues, which could form disulfide bonds and interfere with binding to MHC molecules. An *in silico* algorithm was used to confirm whether the 13 peptides selected in this way were peptides with high binding affinity to MHC class I or MHC class II.

As a result, by confirming that the selected 13 peptides originated from various tumor-associated antigens and bound specifically to HLA-A02/A24 and HLA-DRB1, the 13 peptides were determined as peptide candidates for inducing immunogenicity. The 13 confirmed peptide sequences were synthesized into peptides by requesting a peptide synthesis company (Anygen, Korea).

### <1-2> Confirmation of immunogenicity of selected peptides and selection of final peptides

To confirm the immunogenicity of the 13 peptides selected in Example 1, interferon gamma(IFNg)-enzyme linked immuno-SPOT assay (ELISPOT) was performed.

This experiment is a sensitive immunoassay that can detect cytokines secreted by a single cell. By attaching the cytokines secreted when cells are activated to a capture antibody, the secreted cytokines can be measured without being affected by proteins expressed on the cell surface or proteolytic enzymes. After attaching the cytokine to the capture antibody, the cells are removed, a detecting antibody conjugated to an enzyme that can recognize the attached capture antibody is added, and a chromogenic substrate for the enzyme is added, forming a chromogenic spot at the location of the cell secreting the cytokine, and the number of spots can be used to determine the number of cells that have responded to the stimulating antigen.

The peripheral blood mononuclear cells (PBMCs) required for the experiment were isolated from human venous blood as follows. The venous blood was diluted with 1X PBS, placed in SepMate-50 tubes, and centrifuged at 800 g for 20 minutes at room temperature. After centrifugation, the plasma and the separated PBMCs were washed, placed in each well of a 6 well tissue culture plate at a concentration of 1 x 10⁶ cells/mL, and cultured overnight in an incubator (37°C, 5% CO₂). The next day, the PBMC culture solution was recovered and centrifuged at 2000 rpm for 15 minutes at room temperature. After centrifugation, PBMCs were seeded in a 6 well cell culture plate, and peptides were added thereto and cultured to stimulate the cells.

At this time, the 13 peptides selected in Example 1 were treated alone or with various peptide combinations, and an untreated group and a ConA group were also prepared for comparative analysis. After culturing in a 6-well cell culture plate, 200 µL of the PBMC culture solution was dispensed into each well of the ELISPOT plate and cultured for 24 hours at 37°C and 5% CO₂ conditions. After culture, the plate was washed five times with 1 X PBS to prepare for ELISPOT assay. The detection antibody (R4-642-biotin) was diluted in PBS (0.5% FBS) and added to the plate at 100 µL/well, followed by reaction at room temperature for 2 hours. The plate was then washed 5 times with 1 X PBS, and streptavidin-HRP diluted in PBS (0.5% FBS) was added to the plate at 100 µL/well, followed by reaction at room temperature for 1 h. Then, the plate was washed with 1 X PBS, and TMB substrate solution was added to the plate at 100 µL/well. When the spot color developed clearly, the plate was washed six times with DW to terminate the reaction. After removing the water, the plate was wrapped in foil and dried in the shade for a day. After attaching ELI-FOIL to the dried ELISPOT plate and stamping the membrane, the number of spots in each well was read using an ELISPOT reader. The number of spots in the test group was subtracted from the number of spots in the untreated group to create a graph.

As a result, as shown in Figure 1, it was confirmed that the immunogenicity was increased in the group treated with all the 13 peptides compared to the control group untreated with the peptides. Among them, CHA-6, CHA-10, and CHA-11, which consistently showed the lowest immunogenicity, were excluded. This is because if a peptide showing low immunogenicity is added, the peptide may induce immune tolerance. Therefore, among the 13 peptides, 10 peptides (CHA-1 to CHA-5, CHA-8, CHA-9, CHA-12, CHA-15 and CHA-16) were finally confirmed based on the immunogenicity results (Table 1).

In order to select the peptide combination that can induce the most effective immune response against various cancer antigens among the 10 confirmed peptides, the peptides corresponding to each of the 5 antigens were first mixed and treated to PBMCs of 6 healthy people, and then analyzed the increase and decrease in immunogenicity using ELISPOT assay.

As a result, as shown in Figure 2, it was confirmed that the combination of peptides corresponding to hTERT and WT-1 and the combination of peptides corresponding to MAGE-A3 and survivin enhanced immunogenicity more effectively than the combination of peptides corresponding to five antigens individually. In addition, it was confirmed that when other antigen combinations were added to this combination, or when antigens other than these two combinations were combined, immunogenicity was decreased or increased, but did not increase further than the values of these two combinations.

In the above experiment, there were 6 peptides (4 from hTERT and 2 from WT-1) in the combination of hTERT and WT-1 and 3 peptides (2 from MAGE-A3 and 1 from survivin) in the combination of MAGE-A3 and survivin, so the present inventors performed an experiment to determine whether the immunogenicity increases with the combination of the number of peptides.

For this purpose, the peptides were combined under various conditions as shown in Figure 3 and treated to PBMCs of three healthy people, and the ELISPOT results according to the number of peptides were confirmed.

As a results, as shown in Figure 3, the group treated with 10 peptides showed higher immunogenicity than the groups treated with other combinations, especially than the groups treated with the combination of hTERT+WT-1 and the combination of survivin+MAGE-A3.

Since the combination of 10 peptides (CHA-1, CHA-2, CHA-3, CHA-4, CHA-5, CHA-8, CHA-9, CHA-12, CHA-15, and CHA-16) was composed of the peptides derived from various cancer antigens (hTERT, survivin, MAGE-A3, MUC1, and WT-1), the combination could induce anti-cancer immune responses against various cancer antigens, contained peptides against MHC class I and II that can activate CD8 and CD4 T cells together, which are important for anti-cancer immune responses, and provided the most enhanced immunogenicity. Therefore, the said combination of 10 peptides was confirmed to be the most effective peptide epitope combination for a therapeutic and preventive vaccine against various cancers.

### Example 2: Preparation of anti-cancer vaccines comprising peptides derived from tumor-associated antigens

All peptides were synthesized by standard and well-established solid phase peptide synthesis methods using Fmoc chemistry utilizing a suitable resin from the C-terminus to the N-terminus (Anygen, Korea). The purification and purity of each individual peptide was confirmed by mass spectrometry and HPLC analysis. The peptides were white lyophilized compounds and were obtained with a purity of 95% or higher. All the peptides were used at a concentration of 10 mg/ml.

### Example 3: Preparation of anti-cancer vaccine comprising [peptide derived from tumor-associated antigen] and [adjuvant consisting of lipopeptide and immunoactive substance]

### <3-1 > L-pampo

An anti-cancer vaccine was prepared by mixing the peptide combination selected in Example <1-2> with the Pam3Cys-SKKKK and poly(I:C) adjuvant of the present invention.

First, a test vaccine comprising Pam3Cys-SKKKK and poly(I:C) was prepared to observe the adjuvant effect when formulated using Pam3Cys-SKKKK and poly(I:C) simultaneously. When preparing a vaccine mixture comprising the peptides, 10X buffer (pH 7.0) containing 150 mM NaCl and 10 mM sodium phosphate was used.

### <3-2> Lipo-pam

First, DOTAP:DPPC liposomes were prepared. For this purpose, DOTAP and DPPC were each dissolved in chloroform, and the organic solvent was vaporized with nitrogen while rotating the mixture of DOTAP and DPPC until it was evenly distributed on the walls of the glass container. At this time, a thin film was formed on the wall, and the organic solvent remaining in the formed film was removed by storing it in a vacuum decicator. The completely dried lipid film was rehydrated by adding distilled water. When a multilamella vesicle (MLV) suspension solution was created, 2X buffer (pH 7.0) containing 300 mM NaCl in 20 mM sodium phosphate was added thereto in the same amount as distilled water. The generated MLV was sonicated for 5 cycles of 3 seconds/3 seconds (pulse on/off) for 5 minutes to prepare DOTAP:DPPC liposomes in the form of small unilamellar vesicles (SUVs).

Lipo-pam was then prepared in the same way as the preparation of DOTAP:DPPC liposomes, except that DOTAP, DPPC, and Pam3-CSKKKK were each dissolved using an organic solvent, and then DOTAP and DPPC were mixed and Pam3-CSKKKK was added thereto. A vaccine mixture was prepared by adding the immunoactive substance adjuvant (poly(I:C)) and peptides to Lipo-pam. In this case, 10X buffer (pH 7.0) containing 150 mM NaCl and 10 mM sodium phosphate was used.

### Experimental Example 1: Verification of CD8 T cell-specific immunogenicity induced by peptide combination

CD8+ T cells are the most important immune cells in anti-cancer immunity and directly kill cancer cells and prevent their proliferation. Therefore, if there are many CD8+ T cells specific for tumor-associated antigens, more effective anti-cancer immunity can be induced. In this Example 1, a tetramer binding assay was performed to confirm the proliferation of CD8+ T cells by administration of the peptides presented in Example 1 alone, in combination of the peptides, and in combination with an adjuvant (lipopeptide and poly(I:C)).

The tetramer binding assay is an experimental method that can sensitively verify antigen-specific CD8 T cell responses, especially CTL (cytotoxic T lymphocyte) responses, by measuring the number or frequency (%) of CD8 + T cells to which MHC tetramers are bound. This experiment uses "MHC tetramer," which is four MHC/peptide complexes made into one complex and then attached to fluorophore-labeled streptavidin.

Therefore, in this experiment, the following four tetramers were prepared using the peptides against MHC class I type, that is, HLA-A02 and HLA-24, which can recognize CD8 T cells (CHA-2/HLA-A24 tetramer, CHA-8/HLA-A02 tetramer, CHA-9/HLA-A24 tetramer, and CHA-15/HLA-A02 tetramer).

As in the ELISPOT assay, PBMCs were isolated from human venous blood and dispensed into a 96-well cell culture plate (200 mL/well). The aliquated PBMCs were stimulated by treating with peptides. In this case, each peptide was treated at 5 mg/mL or 50 mg/mL, and the same concentration was used for the combination of 10 peptides confirmed in Examples <1-2>. At this time, a control group in which the peptide was not treated (untreated) or treated with an unrelated peptide (non-specific tetramer) was also prepared for the assay.

The 96-well cell culture plate was placed in an incubator and cultured at 37°C and 5% CO₂ for 24 hours. Upon completion of the culture, the 96-well plate was taken out, the cultured cells were harvested in FACS (fluorescence activated cell sorter) tubes, and centrifuged at 2000 rpm for 15 minutes at room temperature. When centrifugation was completed, the supernatant was removed, FVS (Fixable Viability Stain) was treated, and the cells were resuspended. After resuspension, the FACS tubes were covered with foil and reacted for 15 minutes at room temperature. Each tube was washed with 2 mL of PBS and then centrifuged at 2000 rpm for 5 minutes at room temperature. When centrifugation was completed, the FACS tubes were taken out, the supernatant was removed, each tube was washed with 1 mL of FACS buffer, placed in a centrifuge, and centrifuged at 2000 rpm for 5 minutes at room temperature. When centrifugation was completed, the FACS tubes were taken out, the supernatant was removed, and 100 µL of FACS buffer solution was added to each tube and resuspended. After resuspension, Human Fc R blocking solution and MHC tetramer were added to the FACS tube and mixed well. The FACS tubes were wrapped with foil and reacted at room temperature for 20 minutes. After 20 minutes, anti-human CD3, CD45, and CD8 antibodies were added thereto and mixed well. The FACS tube was wrapped in foil and reacted in a refrigerator, then the FACS tube was taken out of the refrigerator, FACS buffer solution was added, and centrifuged at room temperature. Upon completion of centrifugation, the FACS tubes were taken out, the supernatant was removed, and the cells were resuspended in FACS buffer. Then, a flow cytometer was used to analyze the number of MHC tetramer+CD8+ T cells and the frequency (%) of MHC tetramer+CD8+ T cells in total CD8+ T cells.

As a result, as shown in Figures 4a to 4h, the frequency (%) and total number of tetramer+CD8+T cells specific for each peptide in the group treated with the combination of 10 peptides were significantly higher than the groups treated with CHA-2, CHA-8, CHA-9, and CHA-15 peptides alone (solid box). When the lipopeptide and poly(I:C) adjuvant was mixed with the combination of 10 peptides and treated, it was confirmed that the frequency and number of tetramer + CD8 + T cells for each peptide were increased more than when only the combination of 10 peptides was added (dotted box).

The above results show that the combination of 10 selected peptides could increase CD8+ T cells more effectively than a single peptide that can activate CD8+ T cells, and in particular, when the combination of 10 peptides and the adjuvant were administered together, there was a greater synergistic effect. This is because CD4+ T cells are activated by the peptides that can bind to MHC class II (CHA-3, CHA-4, and CHA-16), especially in the combination of 10 peptides, and subsequently more peptide-specific CD8+ T cells can proliferate with the help of the activated CD4+ T cells. In addition, the synergistic effect on the proliferation of CD8+ T cells can be seen to have been maximized due to the Th1 response induced by the already known lipopeptide and poly(I:C) adjuvant.

### Experimental Example 2: Verification of cytotoxic ability of CD8+ T cells induced by peptide combination

To confirm whether the T cells proliferated and functionally activated by the combination of peptides derived from various tumor-associated antigens could recognize and kill cancer cells as CTLs (cytotoxic T lymphocytes), cytotoxicity analysis was performed in which effector cells and cancer cells were co-cultured and the frequency of cancer cells killed by CTLs was analyzed using a flow cytometer.

First, to prepare effector cells, that is, CTLs proliferated and activated by the peptides, PBMCs were isolated from human venous blood, and then 10 mg/mL of each of the 10 peptides selected in Example <1-2> and 5 ng/mL of hIL-2 were added, followed by culture. As a control, cells cultured with 5 ng/mL of hIL-2 without the peptides were used.

To determine whether the effector cells increased by the peptides can recognize and kill target cancer cells MHC-specifically, they were co-cultured with cancer cells expressing HLA-A02 or HLA-A24 alone, or both HLA-A02 and HLA-A24. Effector cells and target cells (cancer cells) cultured with or without the peptide combination were cultured together at a ratio of 2.5:1, 5:1, and 10:1, respectively, for 16 hours. Then, the extent of cancer cell killing by effector cells was measured using CFSE/7-AAD (carboxyfluorescein succinimidyl ester/7-aminoactinomycin D) staining.

In CFSE/7-AAD staining, target cancer cells were stained with a substance called CFSE, which caused the cancer cells to turn green. The target cancer cells stained with CFSE were cultured with effector cells for 16 hours and then stained again with a substance called 7-AAD. Since 7-AAD stains only dead cells and is colored red, the target cancer cells expressing both CFSE and 7-AAD can be determined as dead cells by effector cells. After staining, the frequency of CFSE+7-AAD+ cancer cells was measured using a flow cytometer, and the results were displayed in a graph.

The experiments were performed targeting SW620, a colon cancer cell line that expresses both HLA-A02 and HLA-A24, PC-3, a prostate cancer cell line that expresses only HLA-24, and MCF-7, a breast cancer cell line and OVCAR-3, an ovarian cancer cell line that express only HLA-02. As a result, it was confirmed that the effector cells activated by the peptide combination killed more cancer cells than the effector cells cultured without the peptides, and the same trend was observed in the three donors. In addition, as the number of effector cells increased, more cancer cells died, confirming the cell-specific cytotoxic effect (Figures 5a to 5h).

### Experimental Example 3: Confirmation of induction of qranzyme B secretion by peptide combination

To confirm whether the T cells proliferated and functionally activated by the combination of peptides derived from various tumor-associated antigens could secrete granzyme B, which can directly kill cancer cells as CTLs, the level of granzyme B in a culture medium in which effector cells and cancer cells were co-cultured was analyzed using ELISA.

Using the same method as in <Example 2> above, the effector cells and target cells (cancer cells) cultured with or without the peptide combination were added at a ratio of 2.5:1, 5:1, and 10:1, respectively, and cultured together for 16 hours to obtain a culture medium. The obtained culture medium was centrifuged at 2000 rpm for 5 minutes, and the supernatant was obtained and used for granzyme B assay.

Granzyme B ELISA was performed using the corresponding protocol using the Human Granzyme B (HRP) (cat no. 3486-1H-6) kit of MABTECH. 2 µg/mL of capture mAb MT34B6 was added to a 96-well immunoplate (100 µL/well) and reacted overnight at 4°C. Upon completion of the reaction, the supernatant was removed and PBST (PBS + 0.05% Tween 20) containing 0.1% BSA was added to the plate at 200 µL/well, followed by reaction for 1 hour at room temperature. The plate was washed five times with PBST (PBS + 0.05% Tween 20), then each supernatant obtained or 0 - 2000 µg/mL of in-kit standard were added to the plate at 100 µL/well and reacted at room temperature for 2 hours. The plate was washed five times with PBST (PBS + 0.05% Tween 20) and then 1 µg/mL of MT28-biotin was added to the plate at 100 µL/well, followed by reaction at room temperature for 1 hour. The plate was washed five times with PBST (PBS + 0.05% Tween 20) and then streptavidin-HRP was added to the plate at 100 µL/well, followed by reaction at room temperature for 1 hour. The plate was washed five times with PBST (PBS + 0.05% Tween 20). TMB substrate was added to the plate at 100 µL/well and reacted at room temperature for 15 minutes, and then 0.2 M sulfuric acid was added thereto at 50 µL/well to stop the TMB substrate reaction. OD₄₅₀ was measured using an ELISA reader, and the amount of granzyme B in the supernatant was analyzed by comparing with a standard material.

As a result, as shown in Figure 6, it was confirmed that significantly higher levels of granzyme B were measured in the culture medium in which effector cells activated by treating PBMCs derived from 6 healthy people with the peptide combination and the colon cancer cell line SW620, prostate cancer cell line PC-3, breast cancer cell line MCF-7, or ovarian cancer cell line OVCAR-3 were co-cultured than in the culture medium in which effector cells without the peptides and cancer cells were co-cultured. Through this, it was found that CTLs recognize cancer cells and secrete granzyme B, thereby inducing the death of cancer cells.

### Experimental Example 4: Verification of cancer cell killing ability of cytotoxic T cells according to cancer antigen or combination of peptide numbers

The CTL activity efficacy of the combination of 10 peptides confirmed to exhibit excellent immunogenicity in Figure 3, and the survivin+MAGE-A3 or hTERT+WT-1 peptide combination showed high immunogenicity in Figure 2, were compared.

The experiment was performed in the same manner as <Experimental Examples 2 and 3> above, and SW620, a colon cancer cell line, was used as the cancer cell.

As a result, as shown in Figures 7a to 7d, it was confirmed that the effector cells activated by treating PBMCs derived from 3 healthy people with the combination of 10 peptides secreted much higher levels of granzyme B and could kill many cancer cells than the effector cells activated by treatment with the survivin+MAGE-A3 or hTERT+WT-1 peptide combination.

The above results suggest that the combination of 10 peptides can induce anti-cancer immune responses against various cancer antigens because it consists of the peptides derived from various cancer antigens (hTERT, Survivin, MAGE-A3, MUC-1 and WT-1), and can activate CD8 and CD4 T cells together, which are important for anti-cancer immune responses, resulting in a high induction of immune responses against cancer cells, and more cancer cells can be killed by CTLs activated through the induced immune response.

### Experimental Example 5: Evaluation of efficacy of inducing cellular immune response of test vaccine prepared with peptide combination and lipopeptide + immunoactive substance adjuvant or other TLR adjuvant in mouse model

The levels of cellular immune response induction of the test vaccine prepared with the combination of 10 peptides and the lipopeptide + immunoactive substance adjuvant (L-pampo) and the test vaccine prepared with the combination of 10 peptides and other TLR adjuvant were compared in a mouse model.

The test vaccines were prepared by mixing 50 µg of each of the 10 peptides and then including the lipopeptide (Pam3Cys-SKKKK) + immunoactive substance (poly(I:C)) adjuvant or other TLR adjuvant (TLR2 ligand lipopeptide, TLR3 ligand poly(I:C), TLR7/8 ligand imiquimod, TLR9 ligand CpG) in the mixture. Each test vaccine was injected subcutaneously into C57BL/6 mice (100 µL/mouse) three times at one-week intervals. One week after the three immunization administrations, the spleen was removed from the mouse, total splenocytes were isolated, and immunogenicity was analyzed using the ELISPOT method.

As a result, as shown in Figures 8a and 8b, it was confirmed that when administering the test vaccine prepared with the Pam3Cys-SKKKK and poly(I:C) adjuvant (L-pampo), the number of cells producing IFN-γ was significantly increased and each peptide-specific secretion of IFN-γ was also increased compared to administering the test vaccine prepared with the TLR2, TLR3, TLR7/8, or TLR9 adjuvant.

The above results show that the anti-cancer vaccine composition provided in one aspect of the present invention can induce a more potent cellular immune response capable of killing cancer cells compared to the vaccine composition comprising the TLR2, TLR3, TLR7/8, or TLR9 adjuvant.

### Experimental Example 6: Evaluation of efficacy of inducing cellular immune response of test vaccine prepared with combination of peptides derived from tumor-associated antigen provided in present invention and lipopeptide + immunoactive substance adjuvant or Lipo-pam adjuvant consisting of liposome with lipopeptide inserted thereon and immunoactive substance

The levels of cellular immune response induction of the test vaccine (Lipo-pam) prepared with the combination of 10 peptides and the lipopeptide + immunoactive substance adjuvant and the test vaccine prepared with the combination of 10 peptides and the Lipo-pam adjuvant consisting of a liposome with a lipopeptide inserted into the surface and an immunoactive substance were compared in a mouse model.

The test vaccines were prepared by mixing 50 µg of each of the 10 peptides and then including the lipopeptide (Pam3Cys-SKKKK) + immunoactive substance (poly(I:C)) adjuvant or the Lipo-pam adjuvant consisting of a liposome with a lipopeptide inserted into the surface and an immunoactive substance (poly(I:C)) in the mixture. Each test vaccine was injected subcutaneously into C57BL/6 mice (100 µL/mouse) three times at one-week intervals. One week after the three immunization administrations, the spleen was removed from the mouse, total splenocytes were isolated, and immunogenicity was analyzed using the ELISPOT method.

As a result, as shown in Figure 9, it was confirmed that when administering the test vaccine prepared with the Lipo-pam adjuvant consisting of a liposome with a lipopeptide (Pam3Cys-SKKKK) inserted into the surface and an immunoactive substance (poly(I:C)), the number of cells producing IFN-γ was significantly increased compared to administering the test vaccine prepared with the Pam3Cys-SKKKK and poly(I:C) adjuvant (Lipo-pam).

The above results show that the lipopeptide and immunoactive substance adjuvant or the Lipo-pam adjuvant consisting of a liposome with a lipopeptide inserted into the surface and an immunoactive substance, the anti-cancer vaccine composition provided in one aspect of the present invention is an anti-cancer vaccine composition that can strongly induce a cellular immune response capable of killing cancer cells.

## Claims

1. An anti-cancer vaccine composition comprising [peptides derived from a tumor-associated antigen (TAA)].

2. An anti-cancer vaccine composition comprising [peptides derived from a tumor-associated antigen (TAA)], and [an adjuvant consisting of a lipopeptide and an immunoactive substance].

3. The anti-cancer vaccine composition according to claim 1 or 2, wherein the tumor-associated antigen is at least one selected from the group consisting of hTERT, MAGE-A3, MUC-1, survivin, and WT-1.

4. The anti-cancer vaccine composition according to claim 1 or 2, wherein the [peptides derived from a tumor-associated antigen] include one or more of the following peptides:
i) Peptide of SEQ. ID. NO: 1 derived from hTERT and of type HLA-A02 (MHC class I) (SEQ. ID. NO: 1: ILAKFLHWL),
ii) Peptide of SEQ. ID. NO: 2 derived from hTERT and of type HLA-A24 (MHC class I) (SEQ. ID. NO: 2: VYAETKHFL),
iii) Peptide of SEQ. ID. NO: 3 derived from hTERT and of type HLA-DRB1 (MHC class II) (SEQ. ID. NO: 3: EARPALLTSRLRFIPK),
iv) Peptide of SEQ. ID. NO: 4 derived from hTERT and of type HLA-DRB1 (MHC class II) (SEQ. ID. NO: 4: RPGLLGASVLGLDDI),
v) Peptide of SEQ. ID. NO: 5 derived from Survivin and of type HLA-A02 (MHC class I) (SEQ. ID. NO: 5: ELTLGEFLKL),
vi) Peptide of SEQ. ID. NO: 8 derived from Mage-A3 and of type HLA-A02 (MHC class I) (SEQ. ID. NO: 8: KVAELVHFL),
vii) Peptide of SEQ. ID. NO: 9 derived from Mage-A3 and of type HLA-A24 (MHC class I) (SEQ. ID. NO: 9: IMPKAGLLI),
viii) Peptide of SEQ. ID. NO: 12 derived from MUC-1 and of type HLA-A02 (MHC class I) (SEQ. ID. NO: 12: TAPPVHNV),
ix) Peptide of SEQ. ID. NO: 15 derived from WT-1 and of type HLA-A02 (MHC class I) (SEQ. ID. NO: 15: RMFPNAPYL), and
x) Peptide of SEQ. ID. NO: 16 derived from WT-1 and of type HLA-DRB1 (MHC class II) (SEQ. ID. NO: 16: KRYFKLSHLQMHSRKH).

5. The anti-cancer vaccine composition according to claim 1 or 2, wherein the [peptides derived from a tumor-associated antigen] recognize MHC class I, MHC class II, or both.

6. The anti-cancer vaccine composition according to claim 2, wherein the adjuvant is prepared by mixing the lipopeptide and the immunoactive substance.

7. The anti-cancer vaccine composition according to claim 2, wherein the adjuvant comprises a liposome with the lipopeptide inserted into the surface of said liposome.

8. The anti-cancer vaccine composition according to claim 2, wherein the lipopeptide is at least one selected from the group consisting of Pam3Cys-SKKKK, Pam3-CSKKKK, PHC-SKKKK, Ole2PamCys-Pam3Cys-SKKKK, Pam3-CSKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, Pam-CSKKKK, Dhc-SKKKK and FSL-1(Pam2CGDPKHPKSF).

9. The anti-cancer vaccine composition according to claim 7, wherein the liposome is composed of lipids.

10. The anti-cancer vaccine composition according to claim 9, wherein the lipid is at least one selected from the group consisting of DOTAP (1,2-dioleoyl-3-trimethylammonium-Propane), DOPE (1,2-dioleoyl-sn-glycero-3-phosphoethanolamine), DDA (dimethyldioctadecylammonium), DC-chol (3β-[N-(N',N'-dimethylaminoethane)-carbamoyl]cholesterol), DOPG (1,2-dioleoyl-sn-glycero-3-[phospho-rac-(1-glycerol)]), DPPC (1,2-dipalmitoyl-sn-glycero-3-phosphocholine), DOPC (1,2-dioleoyl-sn-glycero-3-phosphocholine), and cholesterol.

11. The anti-cancer vaccine composition according to claim 2, wherein the adjuvant is composed of a liposome with a positive charge in which the lipopeptide with a positive charge is inserted into a lipid bilayer of said liposome and the immunoactive substance with a negative charge.

12. The anti-cancer vaccine composition according to claim 2, wherein the immunoactive substance is at least one selected from the group consisting of poly(I:C), QS21, MPLA (monophosphoryl lipid A), CpG, and flagellin.

13. The anti-cancer vaccine composition according to claim 12, wherein the poly(I:C) has a length of 50 to 5,000 bp.

14. The anti-cancer vaccine composition according to claim 6, wherein the lipopeptide and poly(I:C) are mixed in a composition ratio of 1.25:1 to 2:1, wherein the lipopeptide is 0.01 weight% to 0.1 weight%; and the poly(I:C) is 0.01 weight% to 0.08 weight%.

15. The anti-cancer vaccine composition according to claim 1 or 2, wherein the anti-cancer vaccine composition induces humoral immune response and cellular immune response.

16. The anti-cancer vaccine composition according to claim 1 or 2, wherein the anti-cancer vaccine composition enhances Th1 immune response.

17. The anti-cancer vaccine composition according to claim 15, wherein the humoral immune response increases T follicular helper cells (Tfh) to secrete cytokines.

18. The anti-cancer vaccine composition according to claim 15, wherein the cellular immune response enhances secretion of IFN-y, TNF-α, and granzyme B.

19. The anti-cancer vaccine composition according to claim 1 or 2, wherein the composition is an aqueous solution formulation.

20. A pharmaceutical composition for preventing or treating cancer comprising the anti-cancer vaccine composition of claim 1 or 2 as an active ingredient.

21. The pharmaceutical composition for preventing or treating cancer according to claim 19, wherein the cancer is at least one selected from the group consisting of ovarian cancer, benign astrocytoma, malignant astrocytoma, pituitary adenoma, meningioma, cerebral lymphoma, oligodendroglioma, intracranial tumor, ependymoma, brain stem tumor, laryngeal cancer, oropharyngeal cancer, nasal cavity/paranasal sinus cancer, nasopharyngeal cancer, salivary gland cancer, hypopharyngeal cancer, thyroid cancer, oral cancer, thoracic tumor, small cell lung cancer, non-small cell lung cancer, thymic cancer, mediastinal tumor, esophageal cancer, breast cancer, abdominal tumor, stomach cancer, liver cancer, gallbladder cancer, biliary tract cancer, pancreatic cancer, small intestine cancer, colon cancer, anal cancer, bladder cancer, kidney cancer, penile cancer, prostate cancer, cervical cancer, endometrial cancer, uterine sarcoma, vaginal cancer, female external genitalia cancer, and skin cancer

22. The pharmaceutical composition for preventing or treating cancer according to claim 19, wherein the anti-cancer vaccine composition is used in combination with one or more treatments selected from the group consisting of chemotherapy, targeted therapy, immune checkpoint inhibitors, and radiation therapy.

23. A method for preventing, ameliorating or treating cancer, comprising a step of administering the [peptides derived from a tumor-associated antigen (TAA)] to a subject.

24. A method for preventing, ameliorating or treating cancer, comprising a step of administering the [peptides derived from a tumor-associated antigen (TAA)] and the [adjuvant consisting of a lipopeptide and an immunoactive substance].

25. A use of the [peptides derived from a tumor-associated antigen (TAA)] for use in the preparation of a medicament for the prevention, amelioration or treatment of cancer.

26. A use of the [peptides derived from a tumor-associated antigen (TAA)] and the [adjuvant consisting of a lipopeptide and an immunoactive substance] for use in the preparation of a medicament for the prevention, amelioration or treatment of cancer.
